# EUROPEAN PATENT SPECIFICATION

(11) **EP 2 253 316 B1**
(45) Date of publication and mention of the grant of the patent: **14.08.2013**
(21) Application number: 09305464.1
(22) Date of filing: 20.05.2009
(51) Int. Cl.: A61K 31/4178, A61P 1/08, A61K 31/00, A61K 31/4184, A61K 31/437, A61K 31/439, A61K 31/46, A61K 31/4747, A61K 31/496, A61K 31/538, A61K 31/7088, A61K 38/00, A61K 39/395, A61K 48/00

(54) **Serotonin 5-HT3 receptor antagonists for use in the treatment or prevention of an inner ear pathology with vestibular deficits**
Serotonin-5-HT3-Rezeptorantagonisten zur Verwendung bei der Behandlung oder Prävention einer Innenohr-Pathologie mit Vestibularisdefiziten
Antagonistes du récepteur 5-HT3 de la sérotonine pour une utilisation dans le traitement ou la prévention d'une pathologie de l'oreille interne avec déficits vestibulaires

(43) Date of publication of application: 24.11.2010
(73) Proprietor: INSERM (Institut National de la Santé et de la Recherche Médicale), 75013 Paris (FR)
(72) Inventor: Chabbert, Christian, 34295 Montpellier Cedex 05 (FR); Venail, Frédéric, 34295 Montpellier Cedex 05 (FR)
(74) Representative: Verschelde, Claire

(56) References cited:
- US-A- 4 695 578
- US-A- 5 360 800
- US-A- 6 063 802
- US-A1- 2006 074 101
- US-A1- 2007 265 329
- JELLISH W S ET AL: "Ondansetron versus droperidol or placebo when given prophylactically for the prevention of postoperative nausea and vomiting in patients undergoing middle ear procedures." JOURNAL OF CLINICAL ANESTHESIA SEP 1997, vol. 9, no. 6, September 1997 (1997-09), pages 451-456, XP002541970 ISSN: 0952-8180
- RICE G P ET AL: "Ondansetron for intractable vertigo complicating acute brainstem disorders." LANCET 6 MAY 1995, vol. 345, no. 8958, 6 May 1995 (1995-05-06), pages 1182-1183, XP002541971 ISSN: 0140-6736
- MANDELCORN JEFF ET AL: "A preliminary study of the efficacy of ondansetron in the treatment of ataxia, poor balance and incoordination from brain injury." BRAIN INJURY : [BI] OCT 2004, vol. 18, no. 10, October 2004 (2004-10), pages 1025-1039, XP008109968 ISSN: 0269-9052
- HAIN TIMOTHY C ET AL: "Pharmacologic treatment of persons with dizziness." NEUROLOGIC CLINICS AUG 2005, vol. 23, no. 3, August 2005 (2005-08), pages 831-853 , vii, XP008114219 ISSN: 0733-8619
- TSUKAGOSHI S: "[Pharmacokinetics of azasetron (Serotone), a selective 5-HT3 receptor antagonist]" GAN TO KAGAKU RYOHO. CANCER & CHEMOTHERAPY JUN 1999, vol. 26, no. 7, June 1999 (1999-06), pages 1001-1008, XP002553391 ISSN: 0385-0684
- SMITH W W ET AL: "Zacopride, a potent 5-HT3 antagonist." THE JOURNAL OF PHARMACY AND PHARMACOLOGY APR 1988, vol. 40, no. 4, April 1988 (1988-04), pages 301-302, XP002553392 ISSN: 0022-3573
- BROOKES G B: "The pharmacological treatment of Menière's disease", CLINICAL OTOLARYNGOLOGY AND ALLIED SCIENCES, BLACKWELL SCIENCE LTD, OXFORD, GB, vol. 21, no. 1, 1 February 1996 (1996-02-01), pages 3-11, XP002591311, ISSN: 0307-7772

## Description

### FIELD OF THE INVENTION:

The invention relates to a serotonin 5-HT3 receptor antagonist of formula (I) or ondansetron for use in the treatment or prevention of an inner ear pathology with vestibular deficit, as defined in the present claim 1.

### BACKGROUND OF THE INVENTION:

Vestibular (inner ear) deficits can cause dizziness, vertigo, imbalance, hearing changes, nausea, fatigue, anxiety, difficulty concentrating, and other symptoms, with potentially devastating effects on a person's day-to-day functioning, ability to work, relationships with family and friends, and quality of life.

For example, vestibular neuritis is the first cause of hospitalisation for non neurological vertigos. Because its aetiology is largely unknown, epidemiological studies are variable depending on the source (its incidence is believed to be between 3.5 and 50 new cases for 100000 persons / per year). In the past, either an inflammation of the vestibular nerve or labyrinthine ischemia was proposed as a cause of vestibular neuritis. Currently, a viral cause is favoured. A reactivation of herpes simplex virus type 1 would explain the repetition of the vertigo crisis under such a situation.

Vestibular deficits may be also involved in the majority of the fall in the elderly and their prevention became a priority. The fall in the elderly represents indeed more than 1% of the total budget of the health insurance in France (INSEE 1990). It affects in France 30% of people above 65 and 50% above 80. The fall in the elderly is involved in 2/3 of the death caused by accident above 65, and multiplies by 4 the risk of death in the following year.

Current treatments of vestibular deficits mainly focus on reducing the vertigo crisis using vestibuloplegic drugs, while limiting neurovegetative reactions by using anti emetic drugs. Corticosteroids and antiviral drugs are the only medication that tries to limit the spread of vestibular damages in the case of vestibular neuritis (that are assumed to be due to bacterial or virus infections). Their effect remains under debate regarding the lack of aetiology in most vestibular deficits.

For example, recovery after vestibular neuritis is usually incomplete. In a study of 60 patients, horizontal semicircular canal paresis was found in about 90% one month after the onset of symptoms, and in 80% after six months; the caloric responses normalized in only 42%. On the basis of the incidence of this condition, a substantial and permanent unilateral dynamic deficit of the vestibulo ocular reflex, which cannot be compensated for by other mechanisms, develops in approximately 4000 person per year in the United States. This deficit leads to impaired vision and postural imbalance during walking and especially during head movement toward the affected ear.

All below-cited prior art documents disclose ondansetron for the symptomatic treatment of inner ear pathologies with vestibular deficit.

Jellish W S et al: Ondansetron versus droperidol or placebo when given prophylactically for the prevention of postoperative nausea and vomiting in patients undergoing middle ear procedures, Journal of Clinical Anesthesia 1997, vol. 9, no. 6, 451-456

Rice G P et al: Ondansetron for intractable vertigo complicating acute brainstem disorders, Lancet 1995, vol. 345, no. 8958, 1182-1183

Mendelcorn Jeff et al: A preliminary study of the efficacy of ondansetron in the treatment of ataxia, poor balance and incoordination from brain surgery, Brain Injury 2004, vol. 18, no. 10, 1025-1039

Brookes G B et al: The pharmacological treatment of Ménière's disease, Clinical Otolaryngology 1996, vol. 21, no.1, 3-11, discloses ondansetron for the treatment of emesis in Ménière's disease after destructive surgery.

US6063802 can be taken as a closest prior art. It discloses ondasetrone for the treatment of emesis induced by vestibular disorder, such as Ménière's disease.

Accordingly, the opportunity to develop a protective or repair therapy to efficiently rescue the vestibular function represents a relevant issue.

### SUMMARY OF THE INVENTION

The invention relates to a serotonin 5-HT3 receptor antagonist of formula (I) or ondansetron for use in the treatment and/or the prevention of an inner ear pathology with a vestibular deficit as defined in the present claim 1.

### DETAILED DESCRIPTION OF THE INVENTION

A recent clinical investigation carried out by the inventors demonstrated a suitable restorative effect of a serotonin 5-HT3 receptor antagonist (i.e. 1,2,3,9-tetrahydro-9-methyl-3-[(2-methyl-1H-imidazol-1-yl)methyl]-4H-carbazol-4-one, also known as ondansetron) on the vestibular impairments accompanying vestibular neuritis. This serotonin 5-HT3 receptor antagonist efficiently reduces the vestibular deficit encountered under such inner ear pathology. This result is outstanding since it constitutes the first demonstration that a pharmacological therapy focused on the restoration of the vestibular function, may bring concrete solution to rescue vestibular function following vestibular impairments. It also offers a unique opportunity to develop the first curative therapy against vestibular deficits.

Therefore, the present invention provides compositions (such as pharmaceutical compositions) comprising a compound of formula (I) or ondansetron for use in the treatment or prevention of an inner ear pathology with vestibular deficits as defined in the present claim 1.

As used herein, the term "vestibular deficit" refers to a condition characterized by dizziness, visual or gaze disturbances and balance impairment accompanied by neurovegetative expression such as nausea and vomiting. Vestibular deficits could result from diverse damages or dysfunction of the inner ear, among them the acute and chronic syndromes. Examples of inner ear pathologies with vestibular deficits that are contemplated by the invention include vestibular neuritis, Ménière's disease, endolymphatic hydrops, perilymphatic fistula, labyrinthine haemorrhage, chronic or acute labyrinthine infection, serous labyrinthine, barotraumatism, autoimmune inner ear disease, chronic Menière is disease, presbyvestibulia, toxic vestibular impairments.

According to a first aspect, the invention relates to a serotonin 5-HT3 receptor antagonist of formula (I) or ondansetron for use in the treatment or prevention of an inner ear pathology with a vestibular deficit as defined in the present claim 1.

According to a particular embodiment, the serotonin 5-HT3 receptor antagonist for use according to the invention may be a compound of formula (I): 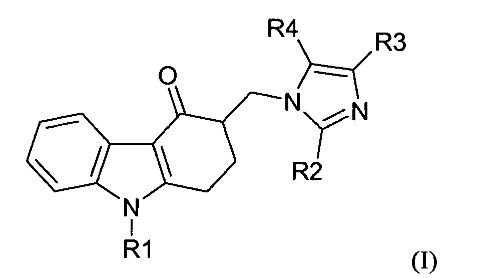 wherein R1 represents a C₃₋₇ cycloalkyl-(C₁₋₄₎ alkyl group or a C₃₋₁₀ alkynyl group; and one of the groups represented by R2, R3 and R4 is a hydrogen atom or a C₁₋₆ alkyl, C₃₋₇ cycloalkyl, C₂₋₆ alkenyl or phenyl-(C₁₋₃) alkyl group and each of the other two groups, which may be the same or different, represents a hydrogen atom or a C₁₋₆ alkyl group; and physiologically acceptable salts and solvates (e.g.hydrates) thereof.

Compounds of formula (I) were described in European Patent n°19156 and in US Patent n°4,695,578.

When the group R1 in general formula (I) represents a C₃₋₇ cycloalkyl-(C₁₋₄) alkyl group, the C₃₋₇ cycloalkyl moiety may be, for example, a cyclopropyl, cyclobutyl, cyclopentyl, cyclohexyl or cycloheptyl group; and the C₁₋₄ alkyl portion may be a methyl, ethyl, propyl, prop-2-yl or butyl group. The group R1 may therefore represent, e.g. a cyclopropylmethyl, cyclooentylpropyl or a cycloheptylmethyl group. When the cycloalkyl ring contains 5, 6 or 7 carbon atoms it may optionally contain one or two double bonds. Examples of such groups include cyclohexenyl and cyclohexadienyl groups.

When R1 represents a C₃₋₁₀ alkynyl group, this may be, for example, a 2-propynyl or 2-octynyl group. It will be understood that when R represents a C₃₋₁₀ alkynyl group, the triple bond may not be adjacent to the nitrogen atom.

Referring to the groups represented by R2, R3 and R4 in general formula (I), an alkyl group may be a straight chain or branched chain alkyl group, for example, a methyl, ethyl, propyl, or prop-2-yl, group; an alkenyl group may be, for example, a propenyl group; a phenyl-(C₁₋₃) alkyl group may be, for example, a benzyl, phenethyl or 3-phenylpropyl group; and a cycloalkyl group may be, for example, a cyclopentyl, cyclohexyl or cycloheptyl group.

It will be appreciated that the carbon atom at the 3- position of the tetrahydrocarbazolone ring is asymmetric and may exist in the R-or S- configuration. Furthermore, it will be appreciated that depending upon the nature of the groups R1, R2, R3 and R4, centres of isomerism may occur elsewhere in the molecule.

In a preferred embodiment, the invention encompasses the use of the optically pure R(+) isomers of compounds of formula (I).

Suitable physiologically acceptable salts of the compounds of general formula (I) include acid addition salts formed with organic or inorganic acids for example, hydrochlorides, hydrobromides, sulphates, phosphates, citrates, fumarates and maleates. The solvates may, for example, be hydrates.

A preferred class of compounds represented by the general formula (I) is that wherein one of the groups represented by R2, R3 and R4 represents a C₁₋₃ alkyl or C₃₋₆ alkenyl group and each of the other two groups, which may be the same or different, represents a hydrogen atom or a C₁₋₃ alkyl group. When R2 represents a hydrogen atom, R3 and/or R4 preferably represents a C₁₋₃ alkyl group. When R2 represents a C₁₋₃ alkyl group R3 and R4 both preferably represent hydrogen atoms.

Preferred compounds of formula (I) may be 1,2,3,9-tetrahydro-3-[(2-methyl-1H-imidazol-1-yl)methyl]-9-(prop-2-enyl)-4H-carbazol-4-one; 9-cyclopentyl-1,2,3,9-tetrahydro-3-[(2-methyl-1H-imidazol-1-yl)methyl]-4H-carbazol-4-one; and 1,2,3,9-tetrahydro-3-[2-methyl-1H-imidazol-1-yl)methyl]-9-(prop-2-yl)-4H-carbazol-4-one and their physiologically acceptable salts and solvates.

A particularly suitable serotonin 5-HT3 antagonist for use according to the invention is Ondansetron®, that is the approved name for 1,2,3,9-tetrahydro-9-methyl-3-[(2-methyl-1H-imidazol-1-yl)methyl]-4H-carbazol-4-one which may be represented by the formula: 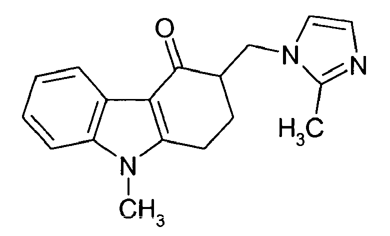 and the physiologically acceptable salts and solvates (e.g. hydrates) thereof.

Another object of the invention relates to a serotonin 5-HT3 receptor antagonist of formula (I) or ondansetron for use in the treatment or prevention of an inner ear pathology with vestibular deficit as defined in claim 1 in a subject in need thereof.

As used herein, the term "subject" denotes a mammal, such as a rodent, a feline, a canine, and a primate. Preferably, a subject according to the invention is a human.

A serotonin 5-HT3 receptor antagonist of formula (I) or ondansetron may be administered in the form of a pharmaceutical composition, as defined below.

Preferably, said antagonist or inhibitor is administered in a therapeutically effective amount.

By a "therapeutically effective amount" is meant a sufficient amount of the serotonin 5-HT3 receptor antagonist of formula (I) or ondansetron to treat or prevent an inner ear pathology with vestibular deficits at a reasonable benefit/risk ratio applicable to any medical treatment.

It will be understood that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

The serotonin 5-HT3 receptor antagonist of formula (I) or ondansetron may be combined with pharmaceutically acceptable excipients, and optionally sustained-release matrices, such as biodegradable polymers, to form therapeutic compositions.

The term "Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The serotonin 5-HT3 receptor antagonist of formula (I) or ondansetron of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active ingredients in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The serotonin 5-HT3 receptor antagonist of formula (I) or ondansetron of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of the invention formulated for parenteral administration, such as intravenous or intramuscular injection, other pharmaceutically acceptable forms include, e.g. tablets or other solids for oral administration; liposomal formulations; time release capsules; and any other form currently used.

In a particular embodiment, the serotonin 5-HT3 receptor antagonist of formula (I) or ondansetron is administered directly in the inner ear through the tympanic membrane. This administration mode may be preferred for introducing a direct and long term effect on the vestibule. Accordingly in a preferred embodiment, the serotonin 5-HT3 receptor antagonist of formula (I) or ondansetron is administered in a gel formulation to allow a long term release of said antagonist or inhibitor in the inner ear.

The invention will be further illustrated by the following figures and examples.

### FIGURES:

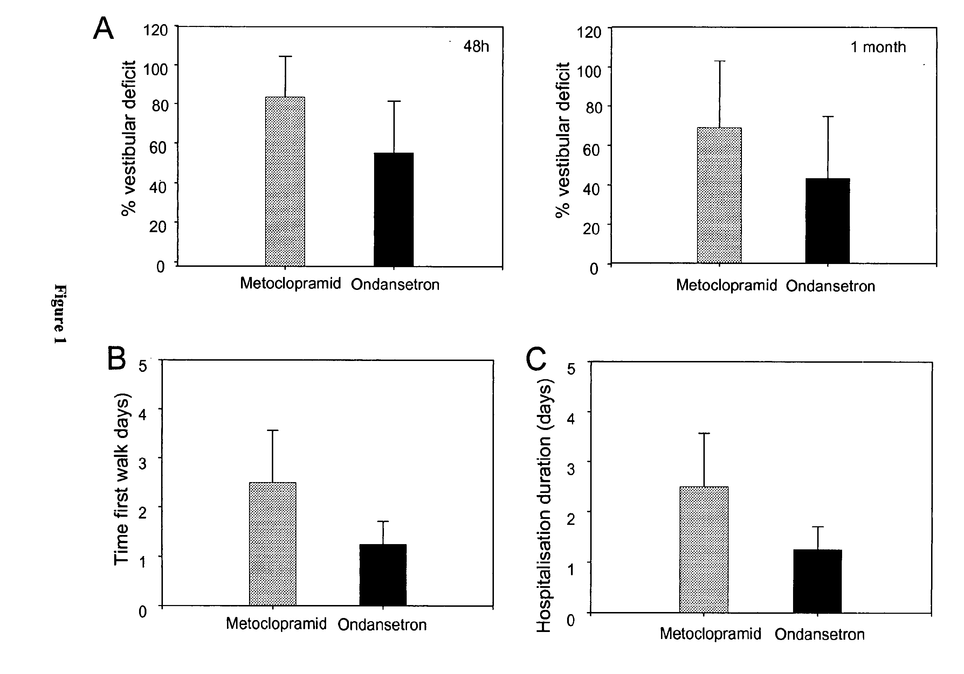

Figure 1: Illustration of the clinical effects of ondansetron versus Metoclopramid in a sample of 20 patients. Illustration of the clinical effects of five days treatment with ondansetron (8 mg/j, n=10) versus Metoclopramid (30 mg/j, n=10) in a sample of 20 patients. Both molecules were administrated together with vestibuloplegics, corticosteroids and antiviral drugs, in patients presenting a suspicion of vestibular neuritis. Vestibulonystagmography was used 48h and 1 month after the treatment (5 days duration) to assess the evolution of the vestibular deficit (A). On the early caloric tests, the vestibular deficit was less pronounced (56,53% versus 84,38%, p=0,03) in patients treated with ondansetron versus metoclopramid. After one month, the difference was not significative anymore on our sample (43,0% ondansetron versus 63,4% metoclopramid, p=0,07). The time of first walk (B) and hospitalisation duration (C) were also significantly reduced in patients administrated with ondansetron.

### EXAMPLES:

The following examples describe some of the preferred modes of making and practicing the present invention. Furthermore, unless the description in an Example is presented in the past tense, the text, like the rest of the specification, is not intended to suggest that experiments were actually performed or data were actually obtained.

### Example 1: reduction of vestibular deficit following acute vestibular neuritis by ondansetron

Methods: A clinical study conducted by the inventors at the Centre Hospitalier Universitaire (CHU) of Montpellier was carried out. Random clinical tests were performed on 20 patients. Patients were selected on the suspicion of vestibular neuritis started for less than 24 h before the hospitalization. The patients were all administered with methylprednisolone and valacyclovir, and either a 5 days treatment with Metoclopramide (30 mg/d, n=10) or with Ondansetron (8 mg/d, n=10). The functional evaluation was based on a early VNG (vestibulonystagmography) test (realized 24 to 48 hours after the beginning of the vestibular deficit) and a VNG at 1 month. The hospitalization duration and the date of the first walk were also recorded.

**Results:** Regarding the early VNG, the vestibular deficit was less pronounced in patients administered with ondansetron (56,53% versus 84,38%, p=0,03). At 1 month, the vestibular deficit did not differed in the two groups of patients (43% O versus 63,4% M, p=0,07). The hospitalization duration was significantly reduced in the ondansetron group (2,88 versus 4,5 days, p=0,03). The time of first walk was also significantly shorter (1,25 versus 2,25 days, p=0,001).

This clinical study demonstrated that ondansetron displays significant effect in reducing the vestibular deficit following acute vestibular neuritis in human (Fig. 1A). Clinical investigations of the vestibular function using caloric testing suggest a direct protective or restorative effect on the peripheral vestibular endorgans. That pharmacological effect does not interfere with the long term central compensation. Its consequence is a significant relieve of the dizziness and a reduction of the hospitalisation duration (Fig. 1B-C). These clinical observations indicate that ondansetron and its derivatives may be useful in the maintenance and restoration of innervation within the vestibule.

### Example 2: The cellular targets of serotonin 5-HT3 antagonists are expressed in the vestibular endorgans

The putative direct effect of serotonin 5-HT3 receptor antagonists on the vestibule is supported by previous report that the serotonin receptors are expressed in the inner ear and specifically in the vestibular endorgans (Johnson and Heinemann, 1995; Gil-Loyzaga et al., 1997), and recent histological experiment from inventors that the 5-HT3 receptors are present in the vestibular epithelia.

### Example 3: Validation of the restorative effect of ondansetron.

Present example intends to validate on animal models of vestibular deficits the restorative effect of a serotonin 5-HT3 receptor antagonist that may selected from the group consisting of ondansetron, granisetron, tropisetron, or palonosetron on the vestibular endorgans observed in human, while determining the biological process (protection / repair) involved in that process. This is assessed by comparing the time courses of histological damages and vestibular deficits in both models of unilateral and bilateral vestibular deficits under early or late application of said serotonin 5-HT3 receptor antagonist. Combination of the two distinct animal models of vestibular deficits allows determining the benefice of using said serotonin 5-HT3 antagonist in conditions of unilateral or bilateral vestibular deficits. To our knowledge, they are the first paradigms focused on the mammal vestibular system. Determination of the biological process involved in the restorative effect of said serotonin 5-HT3 antagonist on the vestibule allows defining the therapeutic window that will be used in future clinical tests.

Validation of the restorative effect of said serotonin 5-HT3 antagonist is assessed by analysing the time course of arrival and removal of both excitotoxic lesions and ensuing vestibular deficit that occur within vestibular endorgans during intra auricular application of glutamate agonists (Brugeaud et al., 2007). In the vestibular endorgans, massive application of kainic acid induces excitotoxic impairments of the neuronal network in the sensory organs (swelling of terminals that contact hair cells). Histological studies of the expansion and reduction of excitotoxic lesions are undertaken using light and electron microscopy. Behavioural evaluation of vestibular deficit is undertaken using specific behavioural tests of the vestibular function. Under the paradigm developed in the rat, a gelfoam containing kainic acid is placed surgically in the middle ear and kainic acid is allowed to diffuse to the inner ear. In most cases, excitotoxic lesions culminate after 48h, and disappear within a week. Protection will be assessed by early administration of said serotonin 5-HT3 antagonist (oral administration of said serotonin 5-HT3 antagonist in drinking water two days before and 3 days during excitotoxicity period); Repair will be assessed upon later administration of said serotonin 5-HT3 antagonist (at the onset of the impairment 48h post excitotoxic chirurgical lesion, Brugeaud et al., 2007). Protection is assessed by early administration of said serotonin 5-HT3 antagonist (oral administration of said serotonin 5-HT3 antagonist in drinking water two days before and 3 days during excitotoxicity period). Repair is assessed upon later administration of said serotonin 5-HT3 antagonist (at the onset of the impairment 48h post excitotoxic chirurgical lesion, Brugeaud et al., 2007).

Validation of the restorative effect of said serotonin 5-HT3 antagonist is also assessed on animal model of bilateral vestibular deficit by analysing the time course of arrival and removal of both excitotoxic lesions and ensuing vestibular deficit following intoxication with nitriles (drinking water delivery - Seoane et al., 2001). Nitrile intoxication induces excitotoxic damages and vestibular deficits identifiable within 3 weeks of chronic intoxication. The damages were reversible in 3 weeks following the removal of intoxication. Protection is assessed by early administration of said serotonin 5-HT3 antagonist (at the start of chronic intoxication); Repair is assessed upon later administration of said serotonin 5-HT3 antagonist (at 3 weeks of chronic intoxication with nitriles, Seoane et al., 2001).

### REFERENCES

Throughout this application, various references describe the state of the art to which this invention pertains.
Brugeaud A, Travo C, Demêmes D, Lenoir M, Llorens J, Puel JL, Chabbert C. Control of hair cell excitability by vestibular primary sensory neurons. J Neurosci. 2007; 27(13):3503-11.
Gil-Loyzaga P, Bartolomé MV, Vicente-Torres MA. Serotonergic innervation of the organ of Corti of the cat cochlea. Neuroreport. 1997;8(16):3519-22.
Johnson DS, Heinemann SF. Embryonic expression of the 5-HT3 receptor subunit, 5-HT3R-A, in the rat: an in situ hybridization study. Mol Cell Neurosci. 1995; 6(2):122-38.
Seoane A, Demêmes D, Llorens J. Relationship between insult intensity and mode of hair cell loss in the vestibular system of rats exposed to 3,3'-iminodipropionitrile. J Comp Neurol. 2001; 439(4):385-99.
Turconi M, Nicola M, Quintero MG, Maiocchi L, Micheletti R, Giraldo E, Donetti A. Synthesis of a new class of 2,3-dihydro-2-oxo-1H-benzimidazole-1-carboxylic acid derivatives as highly potent 5-HT3 receptor antagonists. J Med Chem. 1990 Aug;33(8):2101-8.

## Claims

1. A serotonin 5-HT3 receptor antagonist for use in the treatment or prevention of an inner ear pathology with vestibular deficit, wherein said serotonin 5-HT3 receptor antagonist is selected from the group consisting of
- a compound of formula (I): 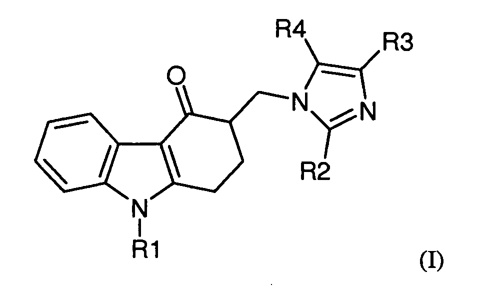 wherein R1 represents a C₃₋₇ cycloalkyl-(C₁₋₄) alkyl group or a C₃₋₁₀ alkynyl group; and one of the groups represented by R2, R3 and R4 is a hydrogen atom or a C₁₋₆ alkyl, C₃₋₇ cycloalkyl, C₂₋₆ alkenyl or phenyl-(C₁₋₃) alkyl group and each of the other two groups, which may be the same or different, represents a hydrogen atom or a C₁₋₆ alkyl group; and physiologically acceptable salts and solvates thereof; and
- ondansetron;
wherein said an inner ear pathology with vestibular deficit is selected from the group consisting of vestibular neuritis, Ménière's disease, endolymphatic hydrops, perilymphatic fistula, labyrinthine haemorrhage, chronic or acute labyrinthine infection, serous labyrinthine, barotraumatism, autoimmune inner ear disease, chronic Menière's disease, presbyvestibulia, and toxic vestibular impairments.

2. The serotonin 5-HT3 receptor antagonist for use according to claim **1**, wherein said serotonin 5-HT3 receptor antagonist is a compound of formula (I): 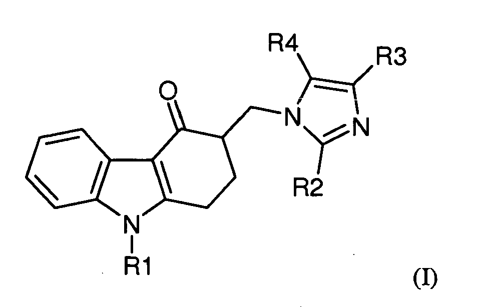 wherein R1 represents a C₃₋₇ cycloalkyl-(C₁₋₄) alkyl group or a C₃₋₁₀ alkynyl group; and one of the groups represented by R2, R3 and R4 is a hydrogen atom or a C₁₋₆ alkyl, C₃₋₇ cycloalkyl, C₂₋₆ alkenyl or phenyl-(C₁₋₃) alkyl group and each of the other two groups, which may be the same or different, represents a hydrogen atom or a C₁₋₆ alkyl group; and physiologically acceptable salts and solvates thereof.

3. The serotonin 5-HT3 receptor antagonist for use according to claim **1**, wherein said serotonin 5-HT3 receptor antagonist is ondansetron.

## Patentansprüche

1. Serotonin 5-HT3-Rezeptorantagonist zur Verwendung bei der Behandlung oder der Vorbeugung einer Innenohrpathologie mit vestibulärem Defizit, wobei der Serotonin 5-HT3-Rezeptorantagnoist ausgewählt ist aus der Gruppe bestehend aus:
- einer Verbindung mit der folgenden Formel (I) 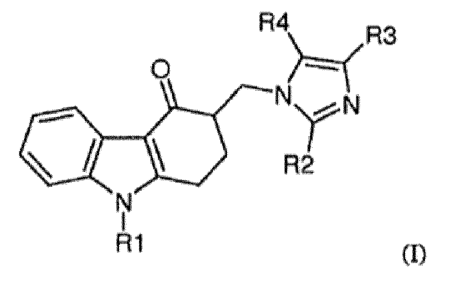 wobei R1 eine C₃₋₇ Cycloalkyl-(C₁₋₄) alkylgruppe oder eine C₃₋₁₀ Alkynylgruppe darstellt; und eine der Gruppen, dargestellt durch R2, R3 und R4, ein Wasserstoffatom oder eine C₁₋₆ Alkyl, C₃₋₇ Cycloalkyl, C₂₋₆ Alkenyl oder Phenyl-(C₁₋₃) alkylgruppe ist und jede der anderen zwei Gruppen, die gleich oder verschieden sein können, ein Wasserstoffatom oder eine C₁₋₆ Alkylgruppe darstellt und physiologisch akzeptable Salze oder Solvate davon; und
- ondansetron;
wobei die Innenohrpathologie mit vestibulärem Defizit ausgewählt ist aus der Gruppe, bestehend aus Neuritis vestibularis, Morbus Ménières, endolymphatischer Hydrops, perilymphatischen Fisteln, Labyrinth-Hämorrhagie, chronische und akute Labyrinth-Infektion, Serumlabyrinth, Barotrauma, autoimmune Innenohrkrankheit, chronischer Morbus Ménières, Presbyvestibulitis und toxischen vestibilären Störungen.

2. Serotonin 5-HT3-Rezeptorantagonist zur Verwendung nach Anspruch 1, wobei der Serotonin 5-HT3-Rezeptorantagonist eine Zusammensetzung mit der folgenden Formel (I) ist: 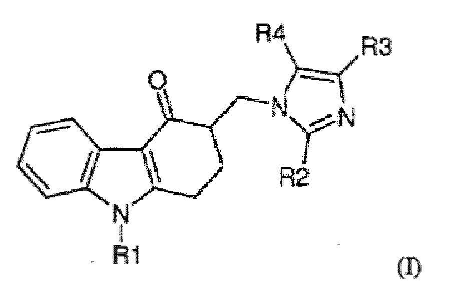 wobei R1 eine C₃₋₇ Cycloalkyl-(C₁₋₄) alkylgruppe oder eine C₃₋₁₀ Alkynylgruppe darstellt; und eine der Gruppen, dargestellt durch R2, R3 und R4, ein Wasserstoffatom oder ein C₁₋₆ Alkyl, C₃₋₇ Cycloalkyl, C₂₋₆ Alkenyl oder Phenyl-(C₁₋₃) alkylgruppe ist und jede der anderen zwei Gruppen, die gleich oder verschieden sein können, ein Wasserstoffatom oder eine C₁₋₆ Alkylgruppe darstellt und physiologisch akzeptable Salze oder Solvate davon.

3. Serotonin 5-HT3-Rezeptorantagonist zur Verwendung nach Anspruch 1, wobei der Serotonin 5-HT3-Rezeptorantagonist Ondansetron ist.

## Revendications

1. Un antagoniste du récepteur 5-HT3 à la sérotonine pour son utilisation dans le traitement ou la prévention d'une pathologie de l'oreille interne avec un déficit vestibulaire, dans lequel ledit antagoniste du récepteur à la sérotonine 5-HT3 est sélectionné parmi le groupe constitué de :
- un composé de formule (I) : 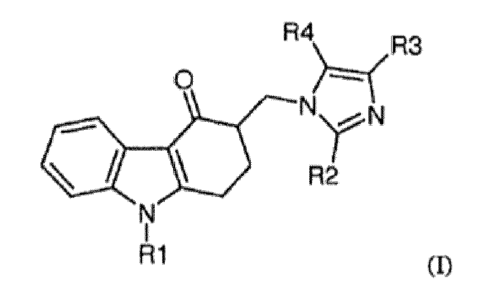 dans lequel R1 représente un groupement (cycloalkyle en C₃ à C₇)-(alkyle en C₁ à C₄) ou un groupement alcynyle en C₃ à C₁₀ ; et un des groupements représentés par R2, R3 et R4 est un atome d'hydrogène ou un groupement alkyle en C₁ à C₆, cycloalkyle en C₃ à C₇, alcényle en C₂ à C₆ ou phényl-(alkyle en C₁ à C₃) et chacun des deux autres groupes, qui peut être le même ou différent, représente un atome d'hydrogène ou un groupement alkyle en C₁ à C₆ ; et des sels et solvates physiologiquement acceptables de celui-ci ; et
- ondansetron ;
dans lequel ladite pathologie de l'oreille interne avec un déficit vestibulaire est sélectionné parmi le groupe constitué de : syndrome vestibulaire, maladie de Ménière, hypertrophie endolymphatique, fistule périlymphatique, hémorragie labyrinthique, infection chronique ou aigue labyrinthique, labyrinthite séreuse, barotraumatisme, maladie autoimmune de l'oreille interne, maladie chronique de Ménière, presbyvestibulie, et déficits toxiques vestibulaires.

2. L'antagoniste du récepteur 5-HT3 à la sérotonine pour son utilisation selon la revendication 1, dans lequel ledit antagoniste au récepteur à la sérotonine 5-HT3 est un composé de formule (I) : 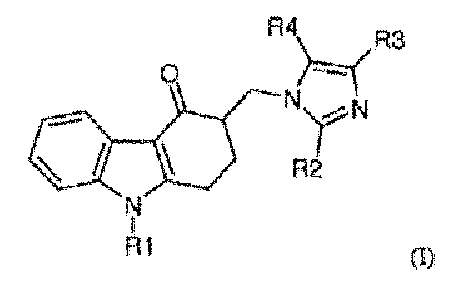 dans lequel R1 représente un groupement (cycloalkyle en C₃ à C₇)-(alkyle en C₁ à C₄) ou un groupement alcynyle en C₃ à C₁₀ ; et un des groupements représentés par R2, R3 et R4 est un atome d'hydrogène ou un groupement alcoyle en C₁ à C₆, cycloalkyle en C₃ à C₇, alcényle en C₂ à C₆ ou phényl-(alkyle en C₁ à C₃) et chacun des deux autres groupes, qui peut être le même ou différent, représente un atome d'hydrogène ou un groupement alkyle en C₁ à C₆ ; et des sels et solvates physiologiquement acceptables de celui-ci.

3. L'antagoniste du récepteur 5-HT3 à la sérotonine pour son utilisation selon la revendication 1, dans lequel ledit antagoniste au récepteur à la sérotonine 5-HT3 est ondansetron.
